# EUROPÄISCHE PATENTSCHRIFT

(11) **EP 0 889 950 B1**
(45) Veröffentlichungstag und Bekanntmachung des Hinweises auf die Patenterteilung: **08.11.2000**
(21) Anmeldenummer: 97916379.7
(22) Anmeldetag: 22.03.1997
(51) Int. Cl.: C12M 1/36

(54) **VERFAHREN ZUR ANALYSE VON EIGENSCHAFTEN EINES BIOLOGISCHEN SYSTEMS**
PROCESS FOR ANALYSING PROPERTIES OF A BIOLOGICAL SYSTEM
PROCEDE POUR L'ANALYSE DES PROPRIETES D'UN SYSTEME BIOLOGIQUE

(30) Priorität: 29.03.1996 DE 19612766
(43) Veröffentlichungstag der Anmeldung: 13.01.1999
(73) Patentinhaber: GfM Gesellschaft für Modulfermenterbau mbH, 58455 Witten (DE)
(72) Erfinder: BARTHOLMES, Peter Private Universität, D-58448 Witten (DE); ROSENTHAL, Werner Private Universität, D-58448 Witten (DE); WOLFF, Elmar Private Universität, D-58448 Witten (DE)
(74) Vertreter: Meyers, Hans-Wilhelm, Dr.Dipl.-Chem.
(86) Internationale Anmeldenummer: EP9701473
(87) Internationale Veröffentlichungsnummer: WO9736993

(56) Entgegenhaltungen:
- WO-A-90/09454
- FR-A- 2 184 035
- FR-A- 2 293 488
- US-A- 2 281 457
- US-A- 4 064 015

## Beschreibung

Gegenstand der vorliegenden Erfindung ist ein Verfahren zur Analyse von Eigenschaften eines biologischen Systems, wobei die Eigenschaften des biologischen Systems durch inhärente Werte, wie Systemparameter und Systemgrößen ausdrückbar sind.

In den letzten Jahren haben sich in zunehmendem Maße biotechnologische Verfahren zur Lösung verschiedenster Problemstellungen als geeignet erwiesen. Bislang war es jedoch häufig dem Zufall überlassen, ob eine bestimmte Eignung eines bestimmten biologischen Systems für die Lösung eines Problems in Frage kam. Wenn beispielsweise die Potenz eines biologischen Systems zum Abbau bestimmter Schadstoffe in Abwässern untersucht werden mußte, konnte aufgrund der Komplexität der Untersuchungsmethoden oft keine eindeutige Aussage getroffen werden, ob der ausgesuchte Mikroorganismus tatsächlich geeignet sein könnte, das zugrundeliegende Problem zu lösen. Desweiteren ist es oftmals schwierig, Änderungen, die das biologische System planmäßig oder ungewollt erfährt, systematisch zu erfassen und gegebenenfalls die durch die Störungen des Systems hervorgerufenen Konsequenzen analytisch zu erfassen um gegebenenfalls gegenzusteuern. Eine Erfassung ist insbesondere dann von Interesse, wenn beispielsweise ein komplexer biotechnologischer Prozeß beherrscht werden soll, oder wenn Bedingungen herausgearbeitet werden sollen, bei denen das biologische System eine Aufgabe in möglichst optimaler Weise oder möglichst optimal angepaßt lösen kann.

Im Stand der Technik wird zur Analyse eines biologischen Systems, insbesondere einer Fermentationskultur, kontinuierlich oder diskontinuierlich eine Probe entzogen. Dann werden die Proben einer Veränderung unterzogen und beobachtet, wie das biologische System darauf reagiert. In der Zwischenzeit verändert sich möglicherweise jedoch der Inhalt des Fermenters, aus dem die Probe entnommen wurde. Dies kann auf z. B. einer induzierten oder spontanen Einstellung der Zellteilung oder Beschleunigung der Zellteilung oder Anschaltung anderer Stoffwechselaktivitäten beruhen, die nicht notwendigerweise in der analysierten oder zu analysierenden Fraktion gleichermaßen erfolgen muß. Aufgrund der recht langwierigen Untersuchungsmethode sind zuverlässige Korrelationen zwischen der Änderung, die das zu untersuchende System zu erfahren hat und deren Auswirkungen und dem jeweiligen Zustand in der Fermentationsausgangslösung, nicht gewährleistet.

Desweiteren sind frühzeitige Erkennungen von sich anbahnenden Änderungen des Zustandes der im Fermenter gezüchteten Kultur oft nicht möglich, so daß erst dann, wenn bereits eine Zustandsänderung beobachtbar eingetreten ist, Maßnahmen ergreifbar werden. Soll beispielsweise eine Kultur von Mikroorganismen daraufhin untersucht werden, ob sie im Stande ist, stickstoffhaltige Abwässer in besonders effizienter Weise abzubauen, muß erst geprüft werden, ob die zur Zeit in Anlage vorhandene Population von Mikroorganismen in der Lage ist, größere Stickstoffmengen zu bewältigen, die möglicherweise spontan im Abwasser erscheinen. Dies ist in aller Regel jedoch nicht in relativ kurzer Zeit meßbar, da erst beobachtet werden muß, ob ein Stickstoffabbau einsetzt und wenn, mit welcher Intensität.

Gleiches gilt, wenn beispielsweise frühe Phasen beginnender Zellteilungsaktivität von Mikroorganismen schnell erfaßt werden müssen. Normalerweise muß abgewartet werden, bis sich die Zahl der Mikroorganismen um ein hinreichendes Vielfaches erhöht hat, was zum Beispiel durch Trübungsmessungen erfaßt werden kann.

Die US-A-4,064,015 betrifft die kontinuierliche Kontrolle von Prozessbedingungen bei Biosynthesen auf der Basis von messbaren Mengen von Stoffen in den Abgasen des Prozesses. Dabei werden Mengen von Stoffen berechnet, die aus diesen Messungen abgeleitet sind, wobei diese gemessenen Mengen für eine Optimierung der Reaktionsbedingung hinsichtlich des Maximums an Output-Rate bestimmter Produkte sorgt. Untersucht werden Systeme zur fermentativen Herstellung von Alkohol. Als berechnete Größen inklusive die zeitliche Ableitung der Geschwindigkeit der Sauerstoffaufnahme werden für eine im wesentlichen kontinuierlichen Kontrolle der Zuführungsgeschwindigkeit bestimmter Nährstoffe oder anderer selektiver Variablen als Mittel zur Optimierung des Prozesses verwendet.

Die FR-A-2,293,488 betrifft die Überwachung und Regelung von Anlagen zur kontinuierlichen Kultur von Mikroorganismen. Dabei wird die Verwendung von Messgrößen aus dem Stoffwechsel der Organismen im Kulturgefäß zur Überwachung und Regelung der Substratzufuhr herangezogen. Als Kenngrößen werden Stoffwechselzwischen- und -endprodukte, Abnahme von Substratkomponenten, Sauerstoffverbrauch und pH-Änderungen angegeben.

Die FR-A-2,184,035 betrifft ein Verfahren zur Regelung und Analyse biochemischer Prozesse sowie eine Anordnung zur Durchführung des Verfahrens. Es werden dabei eine Vielzahl von steuerbaren Variablen und Zustandsvariablen erfasst, wobei jede steuerbare Variable geregelt und innerhalb vorgegebener Toleranzwerte gehalten wird, so dass die vorgebbaren Toleranzwerte im Verlauf des biochemischen Prozesses nachgestellt werden und hierdurch auch die steuerbaren Variablen in jedem Stadium des biochemischen Prozesses zurückwirken. Die erfassten steuerbaren Variablen und Zustandsvariablen auf einer Echtzeit-on-line-Grundlage dienen zur Berechnung von Werten weiterer Zustandsvariablen, die das Stadium des biochemischen Prozesses wiedergeben und eine Regelwirkung auf die steuerbaren Variablen sofort anzeigen. Dadurch werden gewünschte Umgebungsbedingungen in jedem Stadium des biochemischen Prozesses geschaffen.

Das der Erfindung zugrundeliegende technische Problem besteht also unter anderem darin, ein Verfahren anzugeben, mit dem es gelingt, Bewertungen eines komplexen biologischen Systems durchzuführen, in dem bestimmte Größen des Systems erfaßt werden und korreliert werden können, mit Eigenschaften dieses Systems, um gegebenenfalls Prognosen über den Zustand des Systems oder Bewertungen des Systems durchzuführen.

Gelöst wird das der Erfindung zugrundeliegende technische Problem durch ein Verfahren mit den Merkmalen des Anspruchs 1.

Das erfindungsgemäße Verfahren analysiert die Eigenschaften eines biologischen Systems, bei dem die Eigenschaften des biologischen Systems durch inhärente Werte, wie Systemparameter und Systemgrößen ausdrückbar sind. Dabei wird das System durch Änderung vermuteter oder bekannter Systemparameter des biologischen Systems gestört. Es wird dann eine konsistente Beobachtung der Reaktion des Systems auf die Änderung durch Messung mindestens zweier Systemgrößen in mindestens zwei Parallelansätzen und/oder Beobachtungsräumen durchgeführt, so lange sich letztere ändern und/oder keine Änderung der Systemgrößen durch die Störung mehr erfolgt (entsprechend einer Messung vor oder bis zum Erreichen eines Gleichgewichtszustandes des Systems nach der Störung). Dabei tritt mit Änderungs eines Systemparameters die Reaktion des Systems auf diese Änderung ein. Das Verfahren ist mithin ein dynamisches Verfahren. Ausgewertet wird die Dynamik der Reaktion des Systems, hervorgerufen durch die Störung eines Parameters. Hieran schließt sich die Prüfung der Reproduzierbarkeit der durch die Änderung des oder der Systemparameter(s) induzierten Veränderung einer oder mehrerer Systemgrößen an.

Erfindungsgemäß werden die beschriebenen Verfahrensschritte mehrfach wiederholt, mit der Maßgabe, dass jeweils auch nicht untersuchte Systemparameter geändert werden.

Vorzugsweise befindet sich das zu analysierende biologische System vor einer ersten gezielten Änderung eines Systemparameters in einem quasistationären Gleichgewichtszustand bzw. ist die Geschwindigkeit eventueller Änderungen im System konstant oder das biologische System ist in dem untersuchten Bereich, beispielsweise einem Volumenelement in einem Bioreaktor, hinreichend homogen, um eine reproduzierbare Aussage zu gewährleisten.

Erfindungsgemäß lassen sich insbesondere komplexe biologische Systeme untersuchen. Dazu gehören z.B. Population verschiedener Organismen, wie Mikroorganismen, Zellen, Zellkulturen, Zellorganellen etc.

Geänderter Systemparameter und untersuchte Systemgröße können auch identisch sein. Als Systemparameter bzw. Systemgrößen können physikalische, chemische, biologische Zustände oder Erscheinungen, insbesondere solche, mit denen das System mit der Umgebung in Wechselwirkung treten kann oder Kombinationen davon, herangezogen werden. Insbesondere lassen sich als Systemparameter Temperatur, Wärmeinhalte, pH-Werte, Drucke, Partialdrucke von Gasen im biologischen System, Konzentrationen von Substanzen, wie Hormonen oder anderen in das biologische System bildende Medium abgegebene Substanzen, Anzahl biologischer Komponenten, wie Zellen, Zellaggregate, Zellorganellen gleicher oder verschiedener Sorten, heranziehen. Es lassen sich ebenso die Beobachtung des Populationsverhältnisses verschiedener biologischer Komponenten, deren Stoffwechselaktivitäten, Motilitäten, Zellteilungsraten, Änderungen im Zellzyklus, Abgabe von Stoffwechselprodukten oder Änderung des intrazellulären Stoffwechsels, Erscheinen oder Verschwinden von sekundären intra- und/oder extrazellulären Stoffwechselprodukten, wie beispielsweise Mykotoxinen oder Antibiotika zur Erfassung, d. h. Analyse von Eigenschaften eines komplexen biologischen Systems heranziehen. Auch genetische Erscheinungen, wie induzierte oder spontane Mutationen bzw. Variationen können als signifikante Werte zur Bewertung des Zustandes des Komplexen biologischen Systems dienen.

Konsistenz zwischen zwei Datenmengen oder Parametern im Sinne der Erfindung besteht, wenn sie zu einem gemeinsamen zeitlichen und/oder räumlichen Systemzustand gehören. Konsistente Beobachtung der Reaktion des Systems auf die durchgeführte Änderung bedeutet im Sinne der vorliegenden Erfindung auch, daß möglichst gleichzeitig mit der Änderung eines Systemparameters die Reaktion des Systems auf diese Änderung einsetzt. Werden beispielsweise mehrere vergleichende Zyklen des erfindungsgemäßen Verfahrens simultan durchgeführt, in denen zwecks Absicherung statistischer Verhältnisse dieselben Parameter geändert werden, oder aber zur Erfassung möglichst vieler unterschiedlicher Systemparameter durchgeführt, ist es unbedingt erforderlich, eine möglichst zeitnahe Beobachtung durchzuführen, um eine verläßliche Korrelation der in den unterschiedlichen Zyklen des erfindungsgemäßen Verfahrens, die zeitlich parallel laufen, zu ermöglichen.

Außerdem ist es durch die konsistente Erhebung der Daten erst möglich, zeitlich miteinander in Verbindung stehende Prozesse (nicht unbedingt zeitgleiche) zu ermitteln, und so dynamische Modelle für das biologische System zu erstellen.

Die Durchführung der konsistenten Beobachtung erfolgt erfindungsgemäß in mindestens zwei Parallelansätzen und/oder Beobachtungsräumen. Hier kommen als besonders geeignete Vorrichtungen sogenannte Parallelfermenter in Frage, bei denen in mehreren Kammern die komplexen biologischen Systeme, die aus einer authentischen Probe abgeleitet sind, auf ihre Reaktion hinsichtlich der Änderung von Systemparametern untersucht werden.

Erfindungsgemäß bevorzugt ist mithin ein Verfahren, bei dem die konsistente Beobachtung in einer Anordnung von mindestens zwei parallel unter sonst gleichen Bedingungen geführten Fermentern erfolgt.

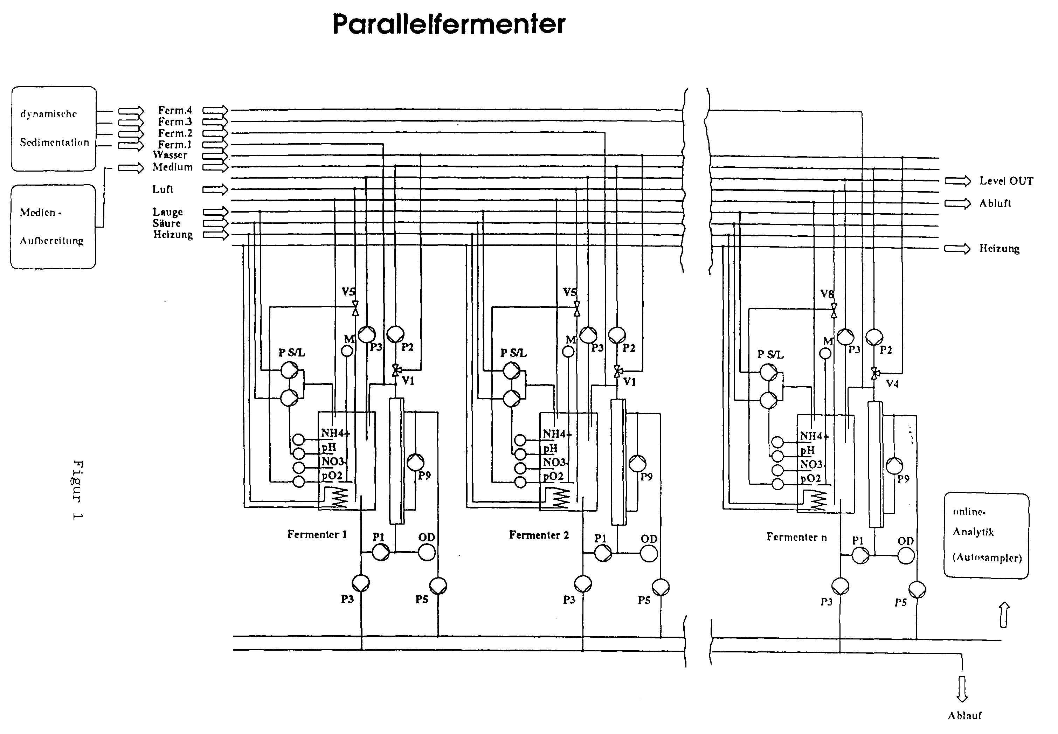

In Fig. 1 ist eine typische Parallelfermenterkonstruktion zum Messen von konsistenten Zuständen eines biologisch Systems dargestellt. In diesem Falle ist der Fermenter auf das Messen von Nitrifiziererleistung mikrobiologischer Systeme spezialisiert. Der Parallelfermenter besteht aus mindestens zwei Fermentereinheiten.

### Allgemein hat ein solcher Parallelfermenter

1. die Eigenschaft prinzipiell während paralleler Fermentation die Bedingungen so gleich wie möglich zu halten.
2. Unabhängig von einzelnen Fermentereinheiten in jeweils einzelnen die veränderbaren Parameter gezielt dynamisch computergesteuert zu modifizieren und die Antworten auf die verschiedenen Parameteränderungen in den unterschiedlichen Fermentereinheiten mit Hilfe von Sensoren an das übergeordnete Rechnersystem zu übertragen, um dort mittels analytischer Verfahren die-Daten zu bearbeiten.

Jeder einzelne Fermenter oder Fermentereinheit des Parallelfermentersystems entspricht in Aufbau und Funktion dem Prinzip eines Bioreaktors. In den jeweiligen Fermentergefäßen können parallele Proben aus einem Hauptprozeß untersucht werden.

Diese können sowohl möglichst gleichen Bedingungen unterworfen, als auch verschieden behandelt werden. Ebenso ist es mit Hilfe des Parallelfermentersystems möglich, mehrere Proben auf unterschiedliche Weise zu behandeln, während in einem Reaktionsgefäß eine Standardmethode durchgeführt wird um die erhaltenen Ergebnisse aus den behandelten Fermentationen zu verifizieren.

In gleicher Weise können mit dem Parallelfermentersystem auch Proben unterschiedlicher Herkunft unter gleichen Bedingungen fermentiert werden.

Bevorzugt ist dabei, daß die Beprobung konsistent, z. B. möglichst zeitgleich oder zeitnah und aus dem gleichen oder einem nahen Bereich innerhalb des Fermenterinhalts des Hauptprozesses stattfindet.

Fig. 1 zeigt schematisch den Aufbau eines Parallelfermenters.

Die Fermenter des Parallelfermentersystems werden über Sammelleitungen mit den zur Fermentation notwendigen Betriebsstoffen versorgt. Die Betriebsstoffe werden z. B. durch Öffnungen im Fermenterdeckel zugeführt.

Dies können im einzelnen sein Wasser, Medium, Luft, Lauge, Säure. Die Versorgung des Heizsystems mit warmem bzw. gekühltem Wasser erfolgt in ähnlicher Weise. Das Heizsystem ist beispielsweise in Form einer Stahlrohrschleife ausgeführt, welche sich z. B. unmittelbar über dem Boden des Fermentergefäßes befindet.

Die Betriebsstoffe werden vorzugsweise gepumpt, sofern es sich um flüssige Komponenten handelt. Gasförmige Stoffe werden vorteilhafterweise mit Überdruck, durch z. B. Einrichtungen zur Vergrößerung der wirksamen Oberfläche der entstehenden Gasblasen, wie beispielsweise Sinterfritten in die Fermentationsbrühe geleitet.

Ferner werden durch Deckelöffnungen verschiedene Sensoren zur quantitativen Bestimmung unterschiedlicher Inhaltsstoffe in den Reaktionsraum geführt.

Hierzu zählen zum Beispiel pH-Sensoren und pO₂-Sensoren. Diese Sensoren ermöglichen eine exakte Steuerung des pH- bzw. pO₂-Wertes durch Steuerung der Säure- und der Laugepumpe sowie des Gaszustromes.

Die Abluft wird insbesondere über die Deckelöffnungen in eine Sammelleitung geführt und in geeigneter Weise abgeleitet.

Zur Niveauregulierung dienen zwei Rohrleitungen, von denen eine vom Deckel bis in die Nähe des Gefäßbodens reicht, während die zweite in Höhe des gewünschten Flüssigkeitsniveaus endet. Durch geeignete Kombination von Pumpen, ist die kontinuierliche Einhaltung eines bestimmten Flüssigkeitspegels im Fermentationsraum erreichbar.

Als Reaktionsgefäß dient ein unten geschlossener Glaszylinder mit einem Volumen von 1,5 l. Vorzugsweise wird das Fermentationsmedium von oben durch den Deckel mit einem Flügelrührer ständig durchmischt. Diese Durchmischung gewährleistet zum Beispiel eine hohe Meßgenauigkeit durch die Sensoren.

Zur Überwachung des Fortgangs der Fermentation, ist die Kontrolle der Inhaltsstoffe im Fermentationsmedium vorteilhaft. Dies kann zum Beispiel das Organismen enthaltende Medium sein, welches insbesondere nicht steril aus dem ablaufenden Probenstrom entnommen und danach analysiert werden kann. Ebenso ist es möglich, zellfreie Proben aus dem Fermenter zu entnehmen um extrazelluläre Stoffwechselendprodukte oder ähnliches zu quantifizieren. Zu diesem Zweck wird aus dem ablaufenden Medium, z. B. mittels einer Pumpe, ein Teilvolumen entnommen und über eine Filtrationseinheit, wie Cross-Flow-Filtrations-Einheit, geführt, wobei durch Beaufschlagen des Flüssigkeitsstromes mit einem Transmembrandruck, z. B. von 0,5 bar, eine Filtration an der Membran der Cross-Flow-Filtrations-Einheit erreicht wird, die über lange Zeit einen kontinuierlichen, zellfreien Probenstrom gewährleistet.

Das Füllen des Reaktorgefässes mit dem zu untersuchenden Medium geschieht automatisch durch Umschalten verschiedener Magnetventile. Ebenso werden automatische Spülzyklen durchgeführt.

Die Steuerung der Prozesse erfolgt über Sensoren, vorzugsweise selbstregelnde oder computerkontrollierte Sensorstrecken.

Zu diesen Sensoren gehören z. B. pH-Sensoren. Nach einem vom Prozeßleitsystem vorgegebenen Sollwert, regeln diese Sensorstrecken den jeweiligen Meßparameter selbständig, wobei ein Meßverstärker und ein Regler für die Aufbereitung der Daten sorgen.

Die zur Prozeßsteuerung notwendigen Sensoren sind vorzugsweise mit einem Meßumwandler verbunden. Die von diesem Meßumwandler ausgehenden Signale werden z. B. über serielle Schnittstellen an einen Meßrechner weitergeleitet. Dieser Rechner bereitet die ermittelten Daten in geeigneter Weise auf und stellt sie dem übergeordneten Prozeßleitsystem zur Verfügung.

Mit Hilfe der Sensoren kann in einem Prozeßleitrechner eine quasi-Abbildung des Parallelfermentersystems geschaffen werden, welche umso genauer, d. h. schärfer wird, je mehr geeignete Sensoren dem System zur Verfügung stehen.

Ebenso besteht die Möglichkeit, den Parallelfermenter an ein übergeordnetes Modellsystem anzukoppeln. Durch rechnergesteuerte Pumpen kann die Kontrolle der Geschwindigkeit und die Menge der zudosierten Medienkomponenten in der Weise erfolgen, daß der Prozeß entlang eines vorgegebenen Modells geführt wird.

Hierbei können die Medienbestandteile dynamisch an die durch den Prozeß veränderten Bedingungen angepaßt werden.

Aufgrund der konsistent erhobenen Daten können Dynamiken unterschiedlicher Meßparameter zur Beurteilung und zur Klassifizierung von zum Beispiel Nitrifizierern herangezogen werden. Die Konsistenz bedingt weiterhin, daß Prozeßgrößen, welche nicht direkt meßbar sind, aus den erhaltenen Sensorsignalen errechnet werden können. Auf diese Weise kommt man zu völlig neuen Erkenntnissen, welche durch den Einsatz herkömmlicher Meßtechnik nicht zu erzielen wären.

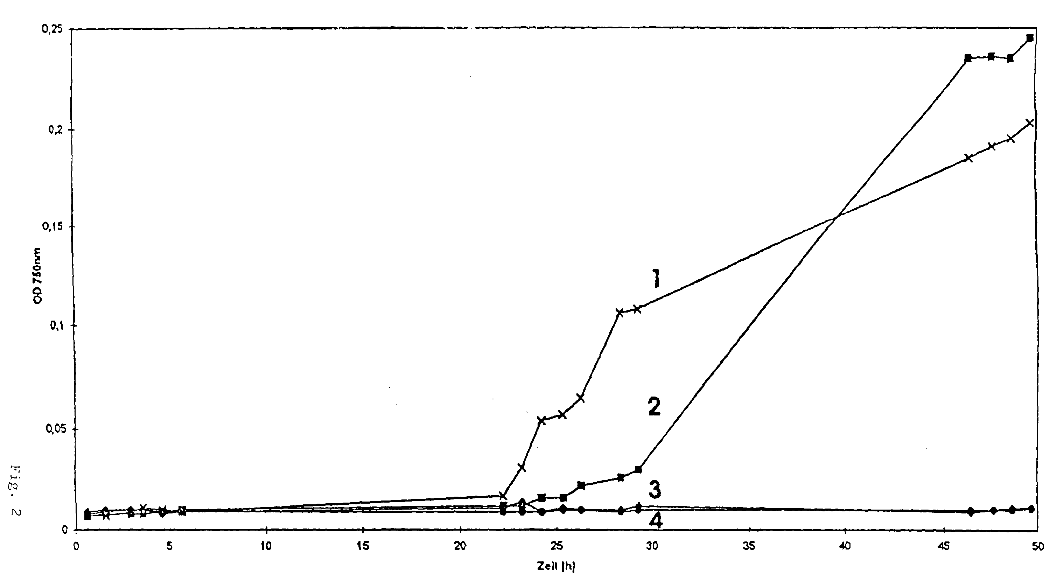

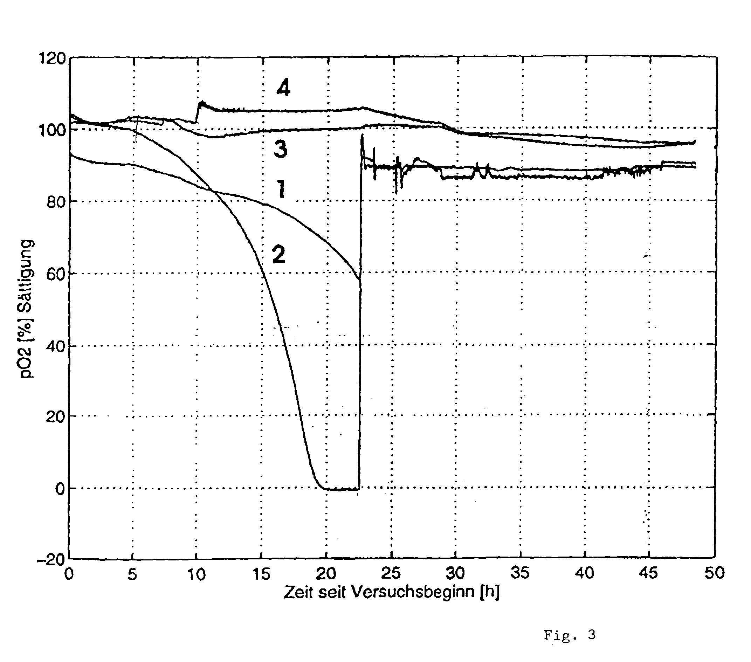

Eine mögliche Methode der Gewinnung von Erkenntnissen ist die gezielte Störung von Gleichgewichten durch pulsweise Zugabe von bestimmten Nährstoffen. Durch die konsequente Beobachtung möglichst vieler verfügbarer Meßgrößen, lassen sich bereits kurze Zeit nach Aufprägen der Störung, anhand der registrierten Anfangsdynamik, Aussagen über z. B. die Leistung bestimmter Organismen im Hinblick auf den Abbau von Inhaltsstoffen von Abwasser treffen (Fig. 2 und Fig. 3).

Jedoch ist der Gleichgewichtszustand nicht zwingend erforderlich. Auch einer bereits vorhandenen Dynamik kann eine Störgröße aufgeprägt werden. Mit Hilfe bestimmter Algorithmen kann das Prozeßleitsystem auch in diesem Fall eine korrekte Bestimmung des Systemzustandes erreichen.

Das erfindungsgemäße Verfahren gewährleistet somit beispielsweise die Erfassung und Bewertung von Abwasserbakterien auf ihre stickstoffmineralisierende Potenz.

Zur Beurteilung der veränderlichen Nitrifiziererleistung im komplexen biologischen System des Rückführungsschlammes einer Kläranlage ist es nötig, hinreichend schnelle Methoden zur Verfügung zu haben. Um Horizonte sedimentierenden Klärschlammes analysieren zu können ist es nötig, parallel Zugriff auf eben diese Horizonte und deren Eigenschaften zu haben. Das Parallelfermentersystem wird gleichzeitig mit entnommener Biomasse aus den verschiedenen Horizonten angeimpft. Da es generell bei dem betrachteten biologischen System um den Abbau von Ammonium bzw. organischem Amin geht, wird dem Parallelfermentersystem der Abbau von definierten Ammoniumpulsen zu intermediärem Nitrit und endgültig Nitrat dynamisch verfolgt und dadurch die Nitrifiziererleistung charakterisiert. Durch dieses Verfähren ist es möglich, innerhalb von wenigen Stunden exakte Aussagen über die Verteilung der Nitrifiziererleistung in den Schlammhorizonten zu machen.

Bei diesem Verfahren wird durch eine mechanische Behandlung von Klärschlammflocken eine bestimmte Art Homogenisierung herbeigeführt, die es erlaubt, in einem nachgeschalteten Sedimentationsfließverfahren nitrifizierende Organismen anzureichern, um diese sodann in den Klärprozeß zurückzuführen. Da die Überprüfung der Qualität von Klassifizierungsmaßnahmen sinnvollerweise nur zeitlich geschehen kann, ist es wichtig, ein konsistentes und schnelles Verfahren zur Bestimmung von Nitrifiziererleistungen zur Hand zu haben. Das geeignete Werkzeug hierfür ist das zuvor beschriebene Parallelfermentersystem. In diesem ist es durch den Vergleich von zeitlichen Abläufen verschiedener Parameter des Stickstoffmetabolismus möglich, die jeweiligen Nitrifiziererleistungen zu bestimmen und zu vergleichen.

Das erfindungsgemäße Verfahren läßt sich zur Bewertung der Durchführbarkeit biotechnologischer Verfahren und/oder deren Optimierung in äußerst vorteilhafter Weise einsetzen.

Beispielsweise läßt sich so auch die Leistung von Mikroorganismen zunächst bewerten und anschließend optimieren. Dazu gehören sowohl solche Organismen, die zum Abbau von problematischen Abfallstoffen aus der Nahrungsmittelindustrie, aus der Tierhaltung, aus der Lederindustrie usw. herangezogen werden, als auch solche, mit deren Hilfe Biochemikalien, Nahrungsstoffe oder Medikamente hergestellt werden können.

Die Erfassung der Reaktion des Systems auf die Änderung vermuteter oder bekannter Systemparameter erfolgt vorzugsweise mittels Datenerfassung durch physikalische, chemische oder biochemische Erfassungsmethoden, wie Spektroskopie, Rezeptorligandwechselwirkung oder Biosensoren, Partialdruckmeßgeräte, Durchflußzytometer, enzymatischer Reaktionen und ähnlichen an sich bekannten Methoden zur Erfassung von Systemgrößen in biologischen Prozessen, insbesondere Fermentationsprozessen.

In Fig. 2 ist dargestellt, wie die Korrelationen zwischen dem Sauerstoffverbrauch und der biologischen Vitalität einer Population von Nitrosomonas europaea, anhand des Sauerstoffpartialdruckes und dessen Änderung in Parallelfermentern analysiert wurden. Die Population, die ein stärkeres Wachstum zeigt, hat auch vorzeitig eine höhere Sauerstoffverbrauchsrate als die, die nur normal wächst. Mit Hilfe einer dynamischen Analyse des Sauerstoffpartialdruckes ist es somit möglich, im Parallelfermenter gezielt verschiedene Wachstumsverhalten von Nitrosomonas europaea zu analysieren.

Die vorliegende Erfindung wird anhand des folgenden Beispiels näher erläutert.

Figur 2 zeigt vier Wachstumskurven von Nitrosomonas europaea in vier Parallelfermentern nach gleichzeitigem Animpfen mit gleicher Konzentration in allen vier Fermentern. Im Fermenter 1 und 2 wurden die gleichen Kulturen wie in 3 und 4, jedoch nach einer kurzen Vorbehandlung, angeimpft. Die Vorbehandlung bestand in einer kurzfristigen Kältebehandlung bei -15°C bis -25°C, insbesondere -20°C. Die Kurven von 3 und 4 zeigen das übliche, langsame Wachstum mit einer Verdopplungszeit von ca. 7 Tagen, während die vorbehandelten Kulturen sich nach 20 Stunden schlagartig vermehren. Diese Wachstumsaktivität ist vorher nicht detektierbar. Wie in Figur 3 dargestellt, läßt sich mit Hilfe von konsistenter Beobachtung metabolischer Parameter prospektiv dieses Verhalten vorhersagen.

Fig. 3 zeigt, daß die Sauerstoffveratmung in Fermenter 1 und Fermenter 2 bereits nach wenigen Stunden das zukünftige Teilungsverhalten ankündigt. Dabei ist sogar die verzögerte Teilung in Fermenter 2, die aber deutlich stärker ist als in Fermenter 1, im dynamischen Sauerstoffverhalten (Änderung der pO₂ (%) Sättigung) repräsentiert. Aus Fig. 2 und 3 folgt, daß durch geeignete Leitparameter und die dazugehörigen prospektiven Computermodelle bei konsistenter Beobachtung (d. h. zeitliche Datenerhebung gleicher Systemzustände) prospektive Steuerung von Hauptprozessen anhand dynamischer Datenerhebung mit Hilfe des zur Messung herangezogenen Parallelfermenters möglich ist.

## Patentansprüche

1. Verfahren zur Analyse von Eigenschaften eines biologischen Systems, wobei die Eigenschaften des komplexen biologischen Systems durch dem biologischen System inhärente Werte, wie Systemparameter und Systemgrößen, ausdrückbar sind, mit den folgenden Verfahrensschritten:
- Störung des biologischen Systems durch Änderung vermuteter oder bekannter Systemparameter des biologischen Systems,
- konsistente Beobachtung der Antwort des biologischen Systems auf die Änderung durch Messung mindestens zweier Systemgrößen, in mindestens zwei Parallelansätzen und/oder Beobachtungsräumen, solange sich die Systemgrößen ändern und/oder bis keine Änderung der Systemgrößen durch die Störung mehr erfolgt,
- wobei mit der Änderung eines Systemparameters die Reaktion des Systems auf diese Änderung einsetzt,
- Prüfung der Reproduzierbarkeit der durch die Änderung des oder der Systemparameter(s) induzierten Veränderung einer oder mehrerer Systemgröße(n) und
- mehrfaches Wiederholen der Verfahrensschritte mit der Maßgabe, dass jeweils noch nicht untersuchte Systemparameter geändert werden.

2. Verfahren nach Anspruch 1, wobei das biologische System Kulturen von Mikroorganismen sind.

3. Verfahren nach Anspruch 1 und/oder 2, wobei der geänderte Systemparameter auch die beobachtete Systemgröße ist.

4. Verfahren nach mindestens einem der Ansprüche 1 bis 3, wobei physikalische, chemische, biologische Zustände oder Erscheinungen oder Kombinationen davon als Systemparameter und/oder Systemgrößen herangezogen werden.

5. Verfahren nach Anspruch 4, wobei die konsistente Beobachtung in einer Anordnung von mindestens zwei parallel unter sonst gleichen Bedingungen geführten Fermentern erfolgt.

## Claims

1. A method for analyzing properties of a biological system, said properties of the complex biological system being expressible by values inherent to the biological system, such as system parameters and system quantities, comprising the following process steps:
- disturbance of the biological system by changing supposed or known system parameters of the biological system;
- consistent monitoring of the response of the biological system to the change by measuring at least two system quantities in at least two parallel runs and/or observation periods as long as the system quantities are changing and/or until a change of the system quantities by the disturbance no longer occurs;
- the response of the system to a change of a system parameter starting with this change;
- checking the reproducibility of the change of one or more system quantities induced by the change of the system parameter(s); and
- repeating the process steps several times with the proviso that each time system parameters are changed which have not yet been changed.

2. The method according to claim 1, said biological system being cultures of microorganisms.

3. The method according to claims 1 and/or 2, the changed system parameter also being the system quantity observed.

4. The method according to at least one of claims 1 to 3, wherein physical, chemical or biological conditions or phenomena or combinations thereof are employed as system parameters and/or system quantities.

5. The method according to claim 4, wherein said consistent monitoring is effected in a set of at least two fermenters run in parallel under otherwise equal conditions.

## Revendications

1. Procédé d'analyse de propriétés d'un système biologique, dans lequel les propriétés du système biologique complexe peuvent s'exprimer par des valeurs inhérentes au système biologique, telles que les paramètres du système et les variables du système, comportant les étapes suivantes :
- perturbation du système biologique par variation de paramètres supposés ou connus du système biologique,
- observation systématique de la réponse du système biologique à la variation, par mesure d'au moins deux variables du système, dans au moins deux essais en parallèle et/ou espaces d'observation, tant que les variables du système varient et/ou jusqu'à ce que ne se produise plus aucune modification des variables du système sous l'effet de la perturbation,
- où, en présence d'une variation d'un paramètre du système, la réaction du système à cette variation se déclenche,
- essai de la reproductibilité de la variation, provoquée par la modification du ou des paramètres du système, d'une ou plusieurs variables du système, et
- répétition multiple des étapes, à la condition que, dans chaque étape, on fasse varier des paramètres du système qui n'ont pas encore été étudiés.

2. Procédé selon la revendication 1, dans lequel le système biologique est constitué de cultures de microorganismes.

3. Procédé selon la revendication 1 et/ou 2, dans lequel le paramètre du système ayant varié est aussi la variable du système telle qu'observée.

4. Procédé selon au moins l'une des revendications 1 à 3, dans lequel on fait appel, en tant que paramètres du système et/ou variables du système, à des états ou phénomènes physiques, chimiques, biologiques, ou leurs combinaisons.

5. Procédé selon la revendication 4, dans lequel l'observation systématique a lieu dans un montage constitué d'au moins deux fermenteurs montés en parallèle, et fonctionnant dans des conditions par ailleurs identiques.
